(19) Europäisches Patentamt
European Patent Office
Office européen des brevets

(11) Veröffentlichungsnummer: **0 080 111**
**B1**

(12) # EUROPÄISCHE PATENTSCHRIFT

(45) Veröffentlichungstag der Patentschrift: **24.10.84**

(51) Int. Cl.³: **C 07 C 125/065**

(21) Anmeldenummer: **82110344.7**

(22) Anmeldetag: **10.11.82**

(54) **Verfahren zur Herstellung von N-aromatisch substituierten Carbamaten aliphatischer oder cycloaliphatischer Alkohole.**

(30) Priorität: **20.11.81 DE 3145926**

(43) Veröffentlichungstag der Anmeldung: **01.06.83 Patentblatt 83/22**

(45) Bekanntmachung des Hinweises auf die Patenterteilung: **24.10.84 Patentblatt 84/43**

(84) Benannte Vertragsstaaten: **BE DE FR GB**

(56) Entgegenhaltungen: **US - A - 2 860 166**

(73) Patentinhaber: **BASF Aktiengesellschaft, Carl-Bosch-Strasse 38, D-6700 Ludwigshafen (DE)**

(72) Erfinder: **Lardon, Hartmut, Dr., Bruesseler Ring 28, D-6700 Ludwigshafen (DE)**
Erfinder: **Seybold, Guenther, Dr., Friedrich-Ebert-Strasse 14, D-6708 Neuhofen (DE)**
Erfinder: **Puster, Peter, Dr., Paracelsussstrasse 8, D-6700 Ludwigshafen (DE)**
Erfinder: **Scheuermann, Horst, Dr., Bexbacher Strasse 41, D-6700 Ludwigshafen (DE)**

**EP 0 080 111 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von N-aromatisch substituierten Carbamaten aliphatischer oder cycloaliphatischer Alkohole durch Umsetzung von aromatischen Säureamiden mit aliphatischen Alkoholen in Gegenwart von Alkalihypochloriten, Wasser und Alkalien und dann Umsetzung des Reaktionsgemisches, das Endstoff und aromatisches Amin enthält, ohne Isolierung des Endstoffs mit Halogenameisensäureester.

N-Arylcarbamate werden üblicherweise durch Umsetzung von Alkoholen mit aromatischen Isocyanaten oder von aromatischen Aminen mit Chlorkohlensäureestern hergestellt (Houben-Weyl, „Methoden der Organischen Chemie", Bd. 8, S. 137 bis 142, Georg Thieme-Verlag, Stuttgart, 1952). Die Synthese der Isocyanate gestaltet sich jedoch im industriellen Massstab sehr aufwendig. Bei beiden Methoden wird die Verfügbarkeit des reinen aromatischen Amins vorausgesetzt. Arylamide, die aus den entsprechenden Carbonsäuren, Carbonsäurechloriden bzw. Anthranilsäuren gut zugänglich sind, lassen sich ebenfalls in N-Arylalkylcarbamate überführen, z.B. durch Umsetzung mit Chlor in einem Alkohol in Gegenwart von Basen (US-PS Nr. 2860166). Das Verfahren beinhaltet jedoch ein hohes Sicherheitsrisiko, da sich Gemische aus Chlor und Alkoholen explosionsartig zersetzen können (Houben-Weyl, loc. cit., Bd. 5/3, S. 600; „Proc. Chem. Soc.", *1963*, S. 213). Weiterhin können Carbamate durch Reaktion des Arylamids mit Brom in einem Alkohol in Gegenwart von entsprechendem Alkalialkoholat (FR-OS Nr. 2369252) hergestellt werden. Diese Variante erfordert den Einsatz von teurem Brom sowie teurem Alkoholat und ist somit im technischen Massstab unwirtschaftlich.

Ein weiteres Verfahren ist die Umsetzung des Amids mit Natriumhypochlorit in einem Eiswasser/Alkohol-Gemisch, in Gegenwart von Natriumhydroxid im Überschuss („J. Med. Chem.", *13*, S. 814 bis 819, 1970). Diese Variante setzt die sicherheitstechnisch harmlosere Chlorlauge ein („Org. Synth.", *49*, S. 9 bis 12, 1969) und erfordert keine aufwendigen technischen Massnahmen. Gegenüber den anderen bekannten Verfahren wird bei dieser Umsetzung kein Arylamid, sondern ein besonders strukturiertes Aralkinylcarbonsäureamid, nämlich das 1-(3,4,5-Trimethoxybenzyl)-3-butinylcarbonsäureamid, als Ausgangsstoff verwendet. Die Ausbeute an Carbamid ist unbefriedigend (50%). Im Falle von Arylamiden gelangt man jedoch nur bei Einsatz von Phenolen als Esteralkohol unter derart milden Bedingungen zu Carbamaten in höheren Ausbeuten (DE-OS Nr. 2548573). Eigene Versuche zeigen, dass auf diesem Weg N-Arylcarbamate aliphatischer Alkohole nur in mässigen Ausbeuten erzielt werden.

Es wurde nun gefunden, dass man N-aromatisch substituierte Carbamate aliphatischer oder cycloaliphatischer Alkohole der Formel:

$$R^1-\underset{\underset{H}{|}}{N}-\overset{\overset{O}{\|}}{C}-OR^2 \qquad (I)$$

worin $R^1$ einen aromatischen Rest und $R^2$ einen aliphatischen oder cycloaliphatischen Rest bedeuten, durch Umsetzung von Säureamiden mit Hypochlorit und Alkoholen vorteilhaft erhält, wenn man

a) aromatische Säureamide der Formel:

$$R^1-\overset{\overset{O}{\|}}{C}-NH_2 \qquad (II)$$

mit aliphatischen oder cycloaliphatischen Alkoholen der Formel:

$$R^2-OH \qquad (III)$$

worin $R^1$ und $R^2$ die vorgenannte Bedeutung besitzen, in Gegenwart von Alkalihypochloriten, Wasser und Alkalien umsetzt,

b) und dann das Reaktionsgemisch, das ein Gemisch von Endstoff I und Amin der Formel:

$$R^1-NH_2 \qquad (IV)$$

worin $R^1$ die vorgenannte Bedeutung besitzt, enthält, ohne Isolierung des Endstoffs mit Halogenameisensäureester der Formel:

$$X-CO_2R^2 \qquad (V)$$

worin $R^2$ die vorgenannte Bedeutung besitzt und X ein Halogenatom bezeichnet, umsetzt.

Die Umsetzung lässt sich bei Verwendung von 3,5-Dichlorbenzamid, Methanol, Natriumhypochlorit und Chlorameisensäuremethylester durch die folgenden Formeln wiedergeben

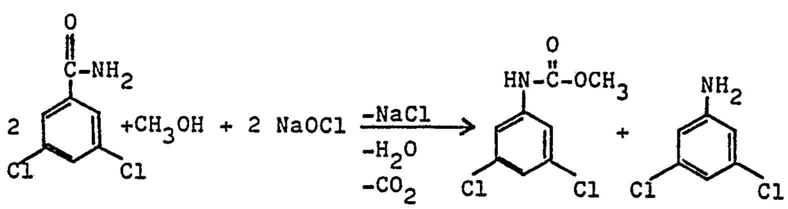


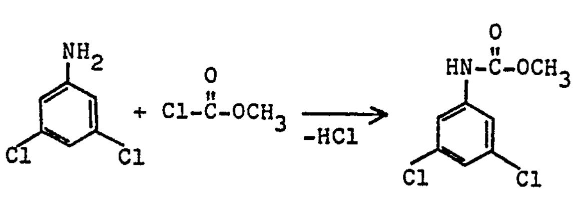

Im Vergleich zu den bekannten Verfahren liefert das Verfahren nach der Erfindung auf einfacherem und wirtschaftlicherem Wege N-aromatisch substituierte Carbamate aliphatischer oder cycloaliphatischer Alkohole in besserer Ausbeute, Reinheit und Raum-Zeit-Ausbeute. Die Ausgangsstoffe sind gut zugänglich. Alle diese vorteilhaften Ergebnisse sind im Hinblick auf den Stand der Technik überraschend. So musste man angesichts der speziellen Substituenten, nämlich den aromatischen Substituenten am Stickstoff und den aliphatischen Substituenten als Esterkomponente zumindest die unbefriedigenden Ausbeuten der bekannten Verfahren erwarten. Darüber hinaus war es überraschend, dass bei der erfindungsgemässen zweistufig einbadigen Arbeitsweise angesichts der zahlreichen reaktiven Gruppen im Gesamtgemisch von a und b nicht eine weit schlechtere Ausbeute und Gemische zahlreicher heterogener Stoffe resultieren.

Bevorzugte Ausgangsstoffe II, III, IV und V und dementsprechend bevorzugte Endstoffe I sind solche, in deren Formeln $R^1$ einen unsubstituierten oder durch Halogenatome, vorzugsweise Chlor- oder Fluoratome, Alkyl- oder Alkoxigruppen mit jeweils 1 bis 4 Kohlenstoffatomen und/oder Nitrogruppen substituierten Phenylrest oder Naphthylrest bedeutet, $R^2$ einen Alkylrest mit 1 bis 10, vorzugsweise 1 bis 4 Kohlenstoffatomen oder einen Cycloalkylrest mit 5 bis 8 Kohlenstoffatomen bezeichnet und X für ein Bromatom oder insbesondere ein Chloratom steht.

So können beispielsweise folgende Ausgangsstoffe II verwendet werden: Benzosäureamid; durch Methyl- Ethyl-, Propyl-, Isopropyl-, Butyl-, Isobutyl-, sek.-Butyl-, tert.-Butyl-, Methoxy-, Ethoxy-, Propoxy-, Isopropoxy-, Butoxy-, Isobutoxy-, sek.-Butoxy-, tert.-Butoxy-, Nitrogruppen, Fluoratome, Chloratome in 2-, 3- und 4-Stellung einfach oder in 3,5-, 2,3-, 2,4-, 2,5-, 2,6-, 3,4-Stellung gleich oder unterschiedlich zweifach am Phenylkern substituiertes Benzoesäureamid.

Folgende Alkohole sind als Ausgangsstoffe III geeignet: Methyl-, Ethyl-, Propyl-, Isopropyl-, Butyl-, Isobutyl-, sek.-Butyl-, tert.-Butyl-, Pentyl-, Hexyl-, Cyclohexyl-, Cyclopentylalkohol.

Beispielsweise kommen folgende Amine IV in Frage: Anilin; mit den schon für die Ausgangsstoffe II verwendeten, vorgenannten Substituienten und in deren Stellungen am Phenylkern substituierte Aniline.

Beispielsweise kommen folgende Halogenameisensäureester V in Frage: Chlorameisensäuremethylester und homologe Ester mit den vorgenannten Alkoholen III; entsprechende Bromameisensäureester.

Als weitere Ausgangsstoffe verwendet man Hypochlorite in wässerigem Medium, in der Regel in Gestalt von entsprechenden wässerigen, alkalischen Lösungen.

Die wässerigen Hypochloritlösungen enthalten im allgemeinen von 5 bis 15, vorzugsweise von 12 bis 14 Gew.-% Hypochlorit. Bevorzugte Alkalihypochlorite sind das Natrium- oder das Kaliumsalz.

Vorteilhaft gelangen wässerige, alkanolische (Ausgangsstoff III) Suspensionen von 1 bis 50 Gew.-% Ausgangsstoff II zur Anwendung. Ausgangsstoffe II und III können mit dem Hypochlorit in stöchiometrischer Menge oder im Überschuss, vorteilhaft in einem Verhältnis von 1,0 bis 1,5, vorzugsweise 1,1 bis 1,2 mol Hypochlorit je Mol Ausgangsstoff II angewendet werden.

Das Wasser kann getrennt von den übrigen Stoffen der Stufe a, zweckmässig aber zusammen mit dem Hypochlorit und/oder den Alkalien dem Ausgangsgemisch zugegeben werden. Vorteilhaft verwendet man im Ausgangsgemisch 15 bis 20, vorzugsweise 17 bis 19 mol Wasser je Mol Ausgangsstoff II und 0,2 bis 1,0, vorzugsweise 0,3 bis 0,6 mol Alkalihydroxid (einschliesslich dem in der Hypochloritlösung enthaltenen Alkali, aber ausschliesslich dem aus dem Alkalihypochlorit stammenden Alkali) je Mol Ausgangsstoff II, wobei das während der Reaktion gebildete Wasser bzw. im Alkalihypochlorit vorhandenes Alkali nicht berücksichtigt werden. Bevorzugt sind Natriumhydroxid und Kaliumhydroxid als Alkalien.

Ausgangsstoffe II und III können in stöchiometrischer Menge oder im Überschuss, vorteilhaft in einer Menge von 1,0 bis 1,5, vorzugsweise von 1,2 bis 1,3 mol III je Mol II umgesetzt werden. Meist wird der eingesetzte Alkohol III gleichzeitig als Reaktionspartner und als Lösungsmittel verwendet und zweckmässig dann in einem Verhältnis von 10 bis 100, insbesondere 20 bis 30 mol je Mol Amid II eingesetzt.

Die Umsetzung wird in beiden Stufen zweckmässig bei einer Temperatur von 0 bis 100° C, vorzugsweise in Stufe a bei 20 bis 80° C, vorzugsweise in Stufe b bei 40 bis 70° C durchgeführt. In einer bevorzugten Ausführungsform erfolgt die Umsetzung der Stufe a zunächst bei Temperaturen von 0 bis 50, insbesondere 18 bis 30° C, anschliessend wird auf 50 bis 100, insbesondere 70 bis 80° C, erhitzt. Beide Stufen können drucklos oder unter Druck, kontinuierlich oder diskontinuierlich durchgeführt werden. Zwar können zusätzlich unter den Reaktionsbedingungen inerte, organische Lösungsmittel verwendet werden, vorteilhaft bildet aber das Ausgangsgemisch, insbesondere der Ausgangsstoff III, das Reaktionsmedium.

Die Umsetzung des Reaktionsgemisches der Stufe a mit dem Ausgangsstoff V kann stöchiometrisch oder im Überschuss von Ausgangsstoff V durchgeführt werden, vorzugsweise in einem Verhältnis von 1,0 bis 1,5, insbesondere 1,2 bis 1,3 mol Ausgangsstoff V je Mol Ausgangsstoff IV.

Die Reaktion kann wie folgt durchgeführt werden: Ein Gemisch aus den Ausgangsstoffen II und III, Hypochlorit, wasser und Alkalien wird während 1,0 bis 3,0 h bei der Reaktionstemperatur der Stufe a gehalten. Dann wird dem Reaktionsgemisch Ausgangsstoff V zugesetzt und das Gemisch der Stufe b während 0,5 bis 1,5 h bei der Reaktionstemperatur der Stufe b gehalten.

In einer bevorzugten Ausführungsform wird das erfindungsgemässe Verfahren so durchgeführt, dass man zunächst das trockene oder Wasserfeuchte Amid II in einem Alkohol III vorlegt, das Alkalihydroxid zugibt, bei 20 bis 30°C Chlorlauge zugibt und die Mischung 10 min bis 1 h bei der gleichen Temperatur rührt. Der Verlauf der Chlorierung des Amids II lässt sich dünnschichtchromatographisch verfolgen. Anschliessend wird das Reaktionsgemisch auf 70 bis 80°C erhitzt. Die Bildung des Gemisches aus Carbamat I und Amin IV lässt sich dünnschichtchromatographisch verfolgen.

Zur Nachacylierung des gebildeten Gemisches werden bevorzugt bei 40 bis 70°C 0,5 bis 0,6 Eq an Halogenameisensäureester V (bezogen auf Ausgangsstoff II) der Reaktionsmischung zugefügt, bis sich dünnschichtchromatographisch kein Amin mehr nachweisen lässt. Die Abtrennung des gebildeten Carbamats I aus dem Reaktionsgemisch kann durch partielles Abdestillieren des eingesetzten Alkohols, Zugabe von Wasser und Absaugen bzw. Extraktion erfolgen.

Falls das Carbamat I weiter umgesetzt werden soll und dabei Isobutanol oder n-Butanol als Lösungsmittel nicht stören, bietet sich als technisch vorteilhafte Aufarbeitung die Extraktion mit einem der genannten Alkohole an. Das Carbamat braucht in diesem Falle nicht isoliert zu werden, sondern kann direkt als Lösung weiterverarbeitet werden. Darüber hinaus kann das Carbamat in dieser gelösten Form einer Reinigung durch Behandeln mit Adsorbentien, wie z.B. Aktivkohle oder Bleicherde, unterworfen werden. Zweckmässig wird dem Endgemisch der Stufe b noch vorhandener Alkohol, z.B. Methanol, entzogen, ein Gemisch von Wasser und Isobutanol (bevorzugtes Verhältnis 0,4 bis 0,8 Teile Wasser je Teil Isobutanol) zugegeben, das Gemisch auf 60 bis 80°C erhitzt und 8 bis 20 min bei dieser Temperatur gehalten. Dann wird vorteilhaft die wässerige Phase abgetrennt und die organische Phase mit heissem (60 bis 80°C) Wasser gewaschen und erneut die wässerige Phase entfernt. Zweckmässig behandelt man die Isobutanolphase bei 60 bis 80°C mit Aktivkohle oder Bleicherde, filtriert und wäscht das Filtergut mit 60 bis 80°C heissem Isobutanol. Man vereinigt jetzt die Isobutanolextrakte und kann Endstoff I in dieser Lösung für andere Umsetzungen verwenden bzw. durch Destillation abtrennen.

Die durch das Verfahren der Erfindung herstellbaren Carbamate I sind wertvolle Ausgangsstoffe für die Herstellung von Farbstoffen, Pharmazeutika und Schädlingsbekämpfungsmitteln. Sie lassen sich beispielsweise durch Reaktion mit 2-Hydroxycarbonsäureestern zu Fungiziden umsetzen (DE-OS Nr. 2813873). Bezüglich der Verwendung wird auf vorgenannte Veröffentlichung verwiesen.

Die in dem folgenden Beispiel genannten Teile bedeuten Gewichsteile.

*Beispiel*

Zu einer Suspension von 391 Teilen feuchten 3,5-Dichlorbenzamids (48,6 Gew.-% Ausgangsstoff II und 51,4 Gew.-% Wasser) in 792 Teilen Methanol gibt man 36 Teile 40gew.-%ige Natronlauge und bei 20°C innerhalb von 15 min 625 Teile Chlorlauge (Gehalt an aktivem Chlor 12,5%). Nach 30minütigem Nachrühren wird das Gemisch innerhalb von 15 min auf 75°C geheizt. Nach 1stündigem Rühren bei dieser Temperatur lässt man das Gemisch abkühlen und gibt bei 60°C innerhalb von 15 min 52 Teile Chlorameisensäuremethylester zu. Unter weiterem allmählichem Abkühlen lässt man das Gemisch 30 min lang nachrühren und destilliert anschliessend innerhalb von 3 h 594 Teile Methanol im Wasserstrahlvakuum ab. Danach werden 200 Teile Wasser sowie 500 Teile Isobutanol zugegeben. Die resultierende Mischung erwärmt man auf 70°C, rührt sie 10 min lang, trennt die wässerige Phase ab, wäscht die organische Phase mit 300 Teilen 70°C heissen Wassers, trennt die wässerige Phase wiederum ab und behandelt die 70°C heisse Isobutanolphase mit 20 Teilen Aktivkohle. Die Aktivkohle wird abfiltriert und viermal mit je 50 Teilen 70°C heissen Isobutanols gewaschen. Die vereinigten Isobutanollösungen enthalten 207 Teile (entsprechend 94% der Theorie) N-(3,5-Dichlorphenyl)-methylcarbamat vom Schmelzpunkt 115 bis 118°C.

## Patentanspruch

Verfahren zur Herstellung von N-aromatisch substituierten Carbamaten aliphatischer oder cycloaliphatischer Alkohole der Formel:

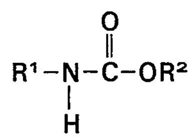
(I)

worin $R^1$ einen aromatischen Rest und $R^2$ einen aliphatischen oder cycloaliphatischen Rest bedeuten, durch Umsetzung von Säureamiden mit Hypochlorit und Alkoholen, dadurch gekennzeichnet, dass man

a) aromatische Säureamide der Formel:

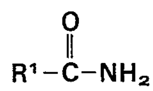
(II)

mit aliphatischen oder cycloaliphatischen Alkoholen der Formel:

$$R^2-OH \qquad (III)$$

worin $R^1$ und $R^2$ die vorgenannte Bedeutung besitzen, in Gegenwart von Alkalihypochloriten, Wasser und Alkalien umsetzt, und

b) und dann das Reaktionsgemisch, das ein Gemisch von Endstoff I und Amin der Formel:

$$R^1-NH_2 \qquad (IV)$$

worin $R^1$ die vorgenannte Bedeutung besitzt, enthält, ohne Isolierung des Endstoffs mit Halogenameisensäureester der Formel:

$$X-CO_2R^2 \qquad (V)$$

worin $R^2$ die vorgenannte Bedeutung besitzt und X ein Halogenatom bezeichnet, umsetzt.

## Claim

A process for the preparation of carbamates of aliphatic or cycloaliphatic alcohols, which carbamates have an aromatic radical attached to the nitrogen atom and are of the formula:

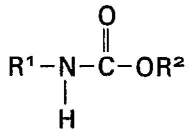 (I)

where $R^1$ is an aromatic radical and $R^2$ is an aliphatic or cycloaliphatic radical, by reacting acid amides with hypochlorite and alcohols, wherein

(*a*) aromatic acid amides of the formula:

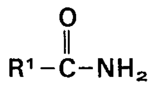 (II)

are reacted with aliphatic or cycloaliphatic alcohols of the formula:

$$R^2-OH \quad (III)$$

where $R^1$ and $R^2$ have the above meanings, in the presence of alkali metal hypochlorites, water and alkalis, and

(*b*) then the reaction mixture containing a mixture of end product (I) and an amine of the formula:

$$R^1-NH_2 \quad (IV)$$

where $R^1$ has the above meaning, is reacted with a halogen formic acid ester of the formula:

$$X-CO_2R^2 \quad (V)$$

where $R^2$ has the above meaning and X is halogen, without isolation of the end product.

## Revendication

Procédé de préparation de carbamates d'alcools aliphatiques ou cycloaliphatiques, substitués sur l'atome d'azote par un groupe aromatique, de formule:

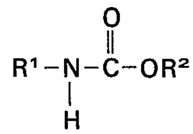 (I)

dans laquelle $R^1$ désigne un groupe aromatique et $R^2$ un groupe aliphatique ou cycloaliphatique, par réaction d'amides d'acides avec un hypochlorite et des alcools, caractérisé en ce que l'on fait réagir

a) des amides d'acides aromatiques de formule:

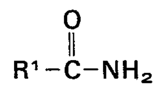 (II)

et des alcools aliphatiques ou cycloaliphatiques de formule:

$$R^2-OH \quad (III)$$

dans lesquelles $R^1$ et $R^2$ possèdent la signification définie, en présence d'hypochlorites de métaux alcalins, d'eau et d'alcalis, et

b) le mélange réactionnel obtenu, qui contient un mélange d'un composé final de la formule (I) et d'une amine de formule:

$$R^1-NH_2 \quad (IV)$$

dans laquelle $R^1$ possède la signification définie, sans isolement du composé final, avec un haloformiate de formule:

$$X-CO_2R^2 \quad (V)$$

dans laquelle $R^2$ possède la signification définie et X désigne un atome d'halogène.